# FASCICULE DE BREVET EUROPEEN

(11) **EP 3 750 687 B1**
(45) Date de publication et mention de la délivrance du brevet: **07.06.2023**
(21) Numéro de dépôt: 20179270.2
(22) Date de dépôt: 10.06.2020
(51) Int. Cl.: B29C 64/118, B29C 64/241, B29C 64/245, B29C 64/40, B29C 64/336, B33Y 10/00, B33Y 30/00, A61F 5/02

(54) **MACHINE D'IMPRESSION 3D À SUPPORT ROTATIF ET PROCÉDÉ DE FABRICATION UTILISANT CELLE-CI**
3D-DRUCKER MIT ROTIERENDER DRUCKUNTERLAGE, UND HERSTELLUNGSVERFAHREN, DAS DIESE NUTZT
MACHINE FOR 3D PRINTING WITH ROTARY SUPPORT AND MANUFACTURING METHOD USING SAME

(30) Priorité: 10.06.2019 FR 1906146
(43) Date de publication de la demande: 16.12.2020
(73) Titulaire: SEGULA ENGINEERING, 92000 Nanterre (FR)
(72) Inventeur: ROUX-MICHOLLET, Jacques, 73000 Jacob Bellecombette (FR); RUSSO, Florence, 69100 Villeurbanne (FR)
(74) Mandataire: IPAZ

(56) Documents cités:
- WO-A1-2018/157253
- CN-A- 106 926 447
- CN-A- 108 245 298
- CN-U- 205 871 201
- JP-A- 2005 138 422
- US-A1- 2016 318 247
- US-A1- 2018 001 560
- US-B2- 9 975 322

## Description

La présente invention concerne d'une part une machine d'impression 3D par dépôt de matière fondue sur un support rotatif, et d'autre part un procédé de fabrication de pièces en matière plastique utilisant ladite machine. La présente invention vise en particulier à fabriquer un corset médical.

Un corset médical est un dispositif médical orthopédique. Il forme un dispositif d'immobilisation ou de soutien rigide, s'étendant autour du tronc de la personne qui le porte, de manière à réaliser un effort de maintien et/ou un effort de correction appliqué sur le tronc de ladite personne. Le corset médical est utilisé par exemple pour soigner ou soulager les pathologies suivantes : douleurs intervertébrales, comme la lombalgie ou la sciatique, des fractures, des tassements vertébraux, ou encore des déformations de la colonne vertébrale du type scoliose idiopathique ou cyphose.

Les corsets médicaux présentent la forme de coques réalisant une paroi continue le long de la peau du patient. Or les corsets médicaux connus sont parfois complexes à adapter à chaque patient, et présentent des inconvénients variés. Par exemple, ils peuvent engendrer des sources de désagréments chez le patient : transpiration excessive, irritation de la peau, apparition d'oedèmes au niveau des os, compression abdominale, problèmes intestinaux, points douloureux au niveau des côtes bloquant la respiration, tendinite. Certains corsets médicaux présentent des ouvertures. Cependant, du fait de l'appui prolongé du corset sur le patient, on assiste à un déplacement et ainsi une mauvaise répartition des graisses du patient.

Il est donc désirable de fournir un corset médical plus facile à fabriquer et à adapter au patient, et offrant un confort amélioré pour le patient tout en améliorant les propriétés mécaniques du corset nécessaires au bon traitement du patient.

De nos jours, les corsets médicaux sont réalisés par moulage de matière plastique, et depuis peu par impression 3D par dépose de filament de matière plastique fondu. Le procédé de fabrication par impression 3D utilise une machine d'impression 3D comprenant un système d'alimentation en filament de polymère, une tête d'impression, dans laquelle le filament est fondu, et un système de guidage afin de déplacer la tête d'impression dans un repère cartésien. Lors du procédé de fabrication, le filament, fondu à la sortie de la tête d'impression, est appliqué sur un plateau rectangulaire horizontal. La pièce désirée est obtenue par empilement vertical de plusieurs couches successives l'une sur l'autre. Le corset présente la forme d'une coque en une ou plusieurs plaques incurvées.

Le procédé de fabrication par impression 3D a pour avantages d'alléger le corset et d'en améliorer l'esthétique.

Cependant, il reste souhaitable de proposer un procédé de fabrication améliorant à la fois les qualités mécaniques, l'allègement et l'esthétique des corsets médicaux. Un but de l'invention est aussi de proposer une machine mettant en oeuvre ledit procédé de fabrication.

On connait du document CN 205 871 201 une machine d'impression 3D conçue pour réaliser des pièces d'avion.

On connait du document US 2018/001560 une autre machine d'impression 3D conçue pour réduire la quantité de résine utilisée pour fabriquer un objet.

Enfin, on connait du document US 2016/318247 une autre machine d'impression 3D conçue pour réaliser des implants à des fins médicales.

### Présentation de l'invention

Selon un premier aspect de l'invention, il est proposé une machine pour impression 3D par dépôt de filament en matière plastique fondu, telle que définie par la revendication 1.

La machine selon l'invention a pour avantages de réaliser des empilements de couches de filaments à la fois verticalement ou horizontalement ou en diagonal. La machine permet ainsi de fabriquer des pièces présentant des propriétés mécaniques localement différentes : par variations de matériau, variations d'épaisseur de matériau, variation de la densité du matériau (à épaisseur donnée) en réalisant un treillis plus ou moins dense (de très écarté à contigu c'est-à-dire matériau plein). Elle permet en particulier de proposer des pièces présentant des propriétés mécaniques localement renforcées du fait de la variation des directions des filaments de chaque couche.

Selon des perfectionnements optionnels de l'invention :
- l'au moins un support présente une surface périphérique continue le long d'une circonférence égale à 360 degrés, ou le long d'un tour complet ou d'une rotation complète,
- l'au moins un support présente une surface périphérique continue le long d'un débattement angulaire inférieur à 360 degrés,
- l'au moins un support ne présente aucune ouverture radiale ou sur sa surface périphérique,
- l'au moins un support présente un axe de rotation incliné d'un angle compris entre 30 degrés et 45 degrés par rapport à la direction l'horizontale,
- l'au moins un support comprend plusieurs supports, interchangeables au sein de ladite machine, présentant des dimensions radiales différentes les uns par rapport aux autres ; cette caractéristique permet de proposer différentes tailles de support en fonction de la taille de la pièce à fabriquer,
- l'au moins un support est un cylindre, de préférence circulaire ou elliptique,
- l'au moins un support comprend un organe chauffant, par exemple par un film polyamide dit « ruban Kapton » ; cette caractéristique permet de faciliter ou d'augmenter l'adhérence du filament sur le support,
- l'au moins un support présente un très bon état de surface, de faibles tolérances de coaxialité, ainsi que de rectitude, cette caractéristique permet de faciliter ou d'augmenter l'adhérence du filament sur le support,
- l'au moins un support est en aluminium, ce matériau a l'avantage de présenter une faible masse volumique, une forte conductivité thermique (dit lambda, par exemple équivalent à 237 W.m-1.K-1) et un coefficient de dilatation raisonnable (par exemple équivalent à 23×10-6 K-1),
- l'au moins un support présente une isolation thermique de façon à isoler thermiquement l'au moins un support du reste de la machine ; en effet sans cela, la chaleur sera transmise aux autres pièces de la machine, ce qui risque de les endommager, et d'autre part, la chaleur serait inutilement perdue,
- l'au moins un support comprend des pièces réalisées dans un matériau isolant thermiquement ou recouvert d'un matériaux isolant,
- la tête d'impression comprend quatre buses agencées et configurées pour distribuer quatre filaments de matières plastiques différentes,
- la tête d'impression comprend quatre buses juxtaposées, de préférence le long d'une droite,
- la tête d'impression comprend au moins deux buses disposées de manière espacée les unes par rapport aux autres, la machine comprenant au moins deux moyens de guidage, chaque moyen de guidage étant associé à une seule buse, de manière que chaque buse puisse se déplacer indépendamment des autres buses,
- la tête d'impression comprend quatre buses disposées de manière espacée les unes par rapport aux autres, la machine comprenant quatre moyens de guidage, chaque moyen de guidage étant associé à une seule buse, de manière que chaque buse puisse se déplacer indépendamment des autres buses,
- la tête d'impression comprend quatre buses espacées les unes des autres et réparties le long de la périphérie de l'au moins un support longitudinalement et/ou circonférentiellement.

Selon un second aspect de l'invention, il est proposé un procédé de fabrication par impression 3D par dépôt de filament en matière plastique fondu, mis en oeuvre par une machine pour impression 3D tel que défini par la revendication 7.

Le procédé selon l'invention permet de réaliser des empilements de couches de filaments à la fois verticalement ou horizontalement ou en diagonal. Le procédé permet ainsi de fabriquer des pièces présentant des propriétés mécaniques localement différentes : par variations de matériau, variations d'épaisseur de matériau, variation de la densité du matériau (à épaisseur donnée). Il permet en outre de réaliser un agencement en forme de treillis plus ou moins dense (de très écarté à contigu c'est-à-dire matériau plein). Il permet en particulier de proposer des pièces présentant des propriétés mécaniques localement renforcées du fait de la variation des directions des filaments entre chaque couche de filaments.

Selon des perfectionnements optionnels de l'invention :
- on met en mouvement de translation selon une direction verticale la tête d'impression par rapport au support de pièce,
- on dépose l'au moins un filament en matière plastique fondu selon une trajectoire prédéterminée sur le support de manière à réaliser une pièce présentant des propriétés mécaniques localement différentes,
- l'au moins un filament en matière plastique fondu est déposé selon une trajectoire prédéterminée sur le support de manière à réaliser une pièce présentant au moins une portion en forme de treillis agencé pour présenter des propriétés mécaniques déterminées localement,
- l'au moins un filament en matière plastique fondu est déposé selon une trajectoire prédéterminée sur le support de manière à réaliser une pièce présentant une épaisseur variable le long de sa périphérie et/ou selon sa direction longitudinale, de façon à présenter des propriétés mécaniques déterminées localement,
- l'au moins un filament en matière plastique fondu est déposé selon une trajectoire prédéterminée sur le support de manière à réaliser une pièce présentant un empilement de couches de filaments juxtaposés, chaque couche présentant une orientation générale des filaments différente de la couche précédente,
- lorsque la machine comprend un dispositif d'alimentation et une tête d'impression agencés et configurés pour alimenter et distribuer au moins deux filaments de matières plastiques, chacun présentant des propriétés mécaniques différentes, on dépose successivement les au moins deux filaments fondus selon une trajectoire prédéterminée sur le support de manière à réaliser une pièce présentant des propriétés mécaniques localement différentes, ou
- lorsque la machine comprend un dispositif d'alimentation et une tête d'impression agencés et configurés pour alimenter et distribuer quatre filaments de matières plastiques, chacun présentant des propriétés mécaniques différentes, on dépose les quatre filaments fondus selon un ordre préétabli et selon une trajectoire prédéterminée sur le support de manière à réaliser une pièce présentant des propriétés mécaniques localement différentes. On entend par trajectoire prédéterminée, la trajectoire de la tête d'impression comprenant les quatre buses juxtaposées, selon un mode de réalisation, ou la trajectoire de chaque buse, chaque buse pouvant se déplacer indépendamment les unes des autres, selon un autre mode de réalisation.

Selon l'un et/ou l'autre des deux derniers modes de réalisations, les matériaux des différents filaments comprennent au moins un matériau support provisoire et un ou plusieurs matériaux constituant la pièce à fabriquer.

Le matériau support provisoire est un matériau sacrificiel. Il peut être dégradable ou soluble dans l'eau.

Le ou les matériaux constituant la pièce à fabriquer est(sont) choisi(s) parmi au moins un matériau favorisant le renfort de la pièce à fabriquer et un matériau favorisant la flexibilité de la pièce à fabriquer. Par exemple, le matériau favorisant le renfort est du type présentant un module d'Young plus grand que celui du matériau favorisant la flexibilité.

De préférence, les matériaux des différents filaments sont choisis parmi du PVA, PLA d'une première composition, PLA d'une seconde composition et PLA d'une troisième composition.

Par exemple, le matériau support provisoire choisi est le PVA car il est d'une grande compatibilité avec le PLA et il présente la particularité d'être soluble dans l'eau. Ainsi, il sera possible d'éliminer le matériau support de la pièce à fabriquer avec un simple passage sous l'eau, sans action d'ébavurage.

Les matériaux constituant la pièce à fabriquer sont par exemple :
- du PLA, ou acide poly(lactique), classique qui servira de base,
- du PLA renforcé qui constituera des parties plus rigides comme des renforts, et
- du PLA flexible qui permettra d'avoir des parties plus souples.

Les matériaux PLA renforcé et/ou PLA flexible sont par exemple des mélanges de matériau PLA et matériau(x) élastomère(s), notamment des mélanges de matériau PLA et matériau PCL (Polycaprolactone) pour obtenir un module d'Young approprié en modulant le taux de mélange.

On entend par PLA classique, un matériau PLA pur présentant un module d'Young prédéterminé, dit moyen.

On entend par PLA renforcé, un matériau PLA pur d'un grade présentant un module d'Young plus grand que le module d'Young du matériau PLA classique et plus grand que le module d'Young du matériau PLA flexible, ou un matériau composite ou un matériau PLA chargé en fibres naturelles.

On entend par PLA flexible, un matériau obtenu par mélange de matériau PLA et matériau élastomère présentant un module d'Young obtenu en fonction du taux de mélange, en particulier des mélanges de matériau PLA et de matériau PCL, les propriétés exactes étant adaptables en modulant le taux de mélange.

De préférence, le procédé de fabrication est utilisé pour fabriquer un corset médical.

### Brève description des dessins

D'autres particularités et avantages de l'invention ressortiront de la description détaillée d'un mode de mise en oeuvre nullement limitatif, et des dessins annexés sur lesquels :
- la Figure 1 est une vue en perspective d'une machine pour impression 3D selon un premier mode de réalisation comprenant un support tournant de pièce à fabriquer dont la surface périphérique est sensiblement elliptique,
- la Figure 2 est une vue de face de la machine de la figure 1,
- la Figure 3 est une vue de dessus de la machine conforme aux figures 1 et 2,
- la Figure 4 est une vue en perspective d'une machine pour impression 3D selon un second mode de réalisation comprenant un support tournant de pièce à fabriquer dont la surface périphérique est sensiblement circulaire,
- la Figure 5 est une vue en perspective de plusieurs couches de filaments, chaque couche présentant des filaments alignés selon une direction qui est différente de celle de la couche adjacente,
- la Figure 6 montre schématiquement trois types de trajectoire qu'au moins un filament peut réaliser sur un support de pièce à fabriquer,
- la Figure 7 est une vue en perspective d'un corset médical présentant une forme générale elliptique et comprenant plusieurs treillis.

Ces modes de réalisations n'étant nullement limitatifs, on pourra notamment considérer des variantes de l'invention ne comprenant qu'une sélection de caractéristiques décrites ou illustrées par la suite isolées des autres caractéristiques décrites ou illustrées (même si cette sélection est isolée au sein d'une phrase comprenant ces autres caractéristiques), si cette sélection de caractéristiques est suffisante pour conférer un avantage technique ou pour différencier l'invention par rapport à l'état de la technique ou pour différencier l'invention par rapport à l'état de la technique antérieure. Cette sélection comprend au moins une caractéristique, de préférence fonctionnelle sans détails structurels, et/ou avec seulement une partie des détails structurels si cette partie uniquement est suffisante pour conférer un avantage technique ou différencier l'invention par rapport à l'état de la technique antérieure.

On va tout d'abord décrire une machine 1 d'impression 3D par dépôt de filament en matière plastique fondu. La machine est en particulier destinée à fabriquer des pièces dont la structure générale est cylindrique, conique et/ou présentant une surface périphérique s'étendant autour d'un axe de rotation de façon que lesdites pièces peuvent présenter, selon un plan transversal, un diamètre variable le long de la circonférence desdites pièces.

Selon n'importe quel mode de réalisation, la machine comprend un support 10 de pièce à fabriquer, ledit support étant rotatif selon un axe de rotation A s'étendant horizontalement ; le support de pièce à fabriquer sera décrit plus en détail par la suite.

Pour ce qui précède et pour la suite de la description, on entend par direction longitudinale une direction parallèle à l'axe de rotation A du support 10. De même, on pourra utiliser une direction axiale, ou une direction dirigée axialement, à la place de la direction longitudinale en évoquant la pièce à fabriquer. En outre, on entend par direction, section ou plan transversal, une direction, une section ou un plan perpendiculaire à l'axe de rotation A. Enfin, on entend par section ou plan longitudinal, une section ou un plan disposé verticalement et dont l'axe de rotation A est confondu avec ladite section ou ledit plan.

La machine 1 comprend un bâti 100 portant tous les éléments de ladite machine et en particulier les éléments nécessaires à la fabrication par impression 3D d'une pièce en matière plastique.

La machine 1 comprend un dispositif d'alimentation 30 en filaments en matière plastique. Le dispositif d'alimentation 30 comprend quatre bobines de filament 31, sur chacune d'elle étant stocké un filament continu enroulé. Chaque bobine 31 comprend un filament constitué d'un matériau spécifique ou d'un matériau d'une composition spécifique, de façon que chaque filament présente des propriétés mécaniques différentes.

De préférence, il est prévu :
- une bobine de filament constitué d'un matériau réalisant un support provisoire ou un support sacrificiel,
- une bobine de filament constitué du matériau acide poly(lactique), dit « PLA » réalisant une partie de base de la pièce à fabriquer,
- une bobine de filament constitué du matériau acide poly(lactique), dit « PLA » réalisant une partie de renfort de la pièce à fabriquer,
- une bobine de filament constitué du matériau acide poly(lactique), dit « PLA » réalisant une partie de flexibilité de la pièce à fabriquer.

Le dispositif d'alimentation 30 comprend au moins un dispositif de dévidement 32 de façon à dévider les quatre bobines de filament 31. Le dispositif de dévidement 32 permet d'acheminer au moins un filament 33 de sa bobine vers une tête d'impression 3D, qui sera décrite par la suite. Selon le mode de réalisation représenté, il est prévu un dispositif de dévidement par bobine 31. Il est par exemple du type système « Bowden ». Ce système est basé sur l'appui d'un pignon d'entrainement et d'un roulement en rotation libre entre lesquels le filament est coincé, permettant la transmission d'une force de poussée en tournant. Le filament est amené de la bobine par une gaine fixée en entrée dudit système et est ensuite conduit dudit système vers la tête d'impression.

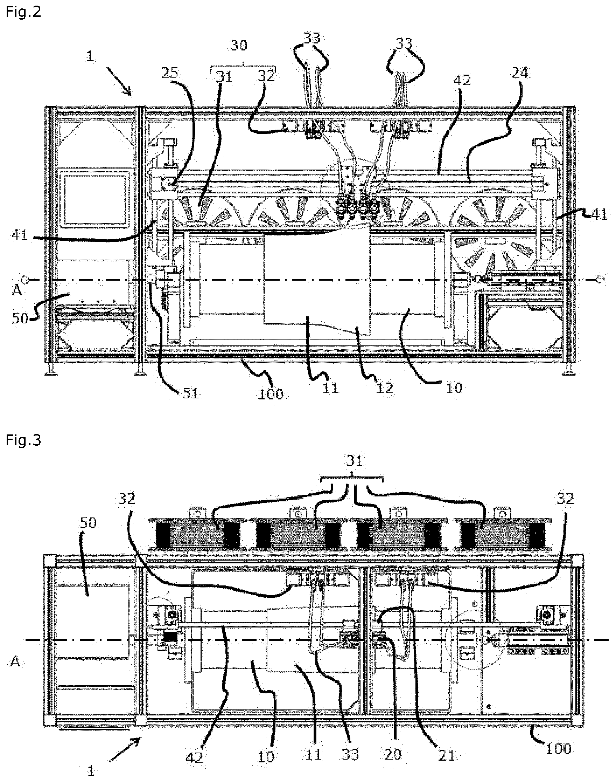

La machine 1 comprend une tête d'impression 20 distribuant chaque filament 33. La tête d'impression comprend autant de buse que de type de filament. En références à la figure 2, la tête d'impression comprend quatre buses 22 juxtaposées les unes par rapport aux autres. Chaque buse comprend un élément chauffant agencé de manière à chauffer un filament en matière plastique à une température supérieure à la température de fusion de ladite matière. Une fois chauffé, le filament fondu sort de la buse dont l'orifice présente un diamètre de 0,3 à 0,5 millimètre.

Selon un mode de réalisation particulier non représenté, les quatre buses sont espacées les unes par rapport aux autres. Leur déplacement s'effectue suivant la direction longitudinale le long de la génératrice du support et est réalisé indépendamment les unes des autres. Les quatre buses sont totalement indépendantes et peuvent déposer le filament individuellement ou simultanément sur cette même génératrice.

La machine comprend des moyens de guidage 40 de la tête d'impression 20 relativement au bâti 100. Ils sont agencés et configurés pour déplacer la tête d'impression au-dessus du support 10 de pièce à fabriquer. En référence à la figure 3, les moyens de guidage sont agencés et configurés pour déplacer la tête d'impression 20 dans le plan longitudinal du support 10. De préférence, les moyens de guidage sont agencés et configurés pour déplacer la tête d'impression 20 uniquement dans le plan longitudinal du support 10. Les moyens de guidage 40 comprennent des moyens de guidage vertical 41 disposés à chaque extrémité axiale du support 10 de pièce à fabriquer. Les moyens de guidage vertical sont des tiges formant des rails verticaux. Les moyens de guidage 40 comprennent des moyens de guidage horizontal 42 s'étendant entre les moyens de guidage vertical 41. Les moyens de guidage horizontal sont des tiges formant des rails horizontaux. Au moins un moteur électrique permet le déplacement vertical des moyens de guidage horizontal relativement aux moyens de guidage vertical.

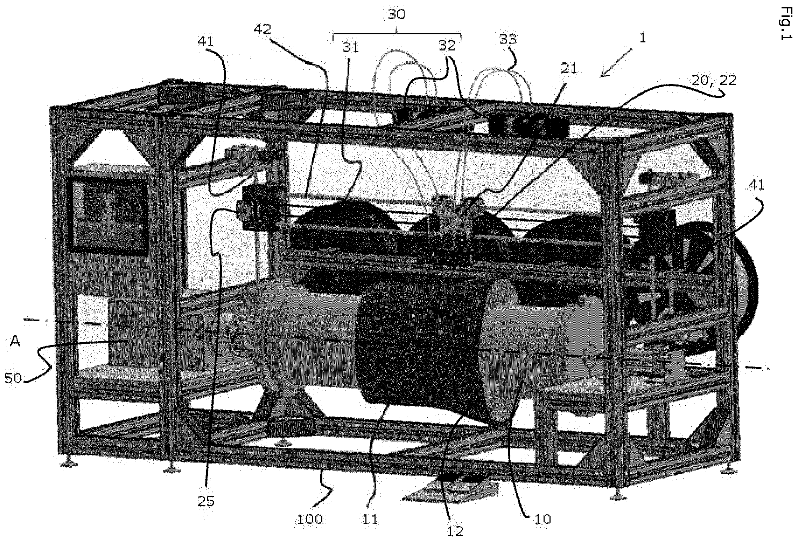

En référence aux figures 1 à 3, la machine comprend des moyens de déplacement de la tête d'impression. Les moyens de déplacement comprennent un convoyeur 21 de tête d'impression portant ladite tête, le convoyeur 21 étant relié en coulissement avec les moyens de guidage horizontal 42. Ils comprennent en outre une courroie 24 et un moteur électrique 25, la courroie 24 étant relié au convoyeur 21 et s'étend le long des moyens de guidage horizontal. Le moteur 25 est disposé sur des moyens de guidage vertical et est relié à la courroie 24 de manière à permettre le déplacement de la tête d'impression 20 au-dessus du support de pièce à fabriquer.

De préférence, les moteurs électriques sont des moteurs pas à pas, qui permettent un positionnement précis.

Selon un premier mode de réalisation et en référence aux figures 1 à 3, la machine comprend un support 10 de pièce à fabriquer, le support 10 présentant une structure générale cylindrique et un axe de rotation A. Le support 10 est disposé relativement au bâti 100 de façon que l'axe de rotation A est disposé horizontalement.

Le support 10 comprend une surface périphérique 11 de travail sur laquelle le ou les filaments en matière plastique fondu(s) est ou sont déposé(s). La surface périphérique 11 s'étend autour de l'axe de rotation A. Elle présente une forme elliptique selon sa section transversale. En outre selon la section longitudinale du support 10, la surface périphérique 11 s'évase près d'une extrémité 12 de ladite surface. Dans le cadre de la fabrication d'un corset médical, une forme elliptique et/ou évasée permet de réduire la consommation de matériau provisoire ou sacrificiel, car ces formes se rapprochent le plus de la morphologie du buste humain.

La machine comprend un moyen de rotation 50 du support 10 de façon que le support de pièce est rotatif selon son axe de rotation A par rapport au bâti 100. Le moyen de rotation comprend un moteur électrique. Selon le mode de réalisation représenté, le moteur électrique 50 est disposé à une extrémité du support 10, de façon que l'arbre moteur 51 est relié directement au support 10. Le support 10 comprend en outre deux éléments d'arbre, chaque élément d'arbre étant disposé à une extrémité du support 10 et étant disposé selon l'axe de rotation A du support 10. L'arbre moteur 51 est alors directement relié à un élément d'arbre.

De préférence, un agencement de pose et dépose rapide est prévu au niveau d'une extrémité du support de pièce à fabriquer. Par exemple, un tel agencement est prévu près d'au moins un élément d'arbre, ou près des deux éléments d'arbres. Selon un mode de réalisation particulier non représenté, le support comprend un accouplement de type cannelé entre le corps du support et un élément d'arbre. Le corps du support présente un alésage d'axe confondu avec l'axe de rotation A, l'alésage comprenant des rainures dirigées axialement de manière à recevoir des rainures associées d'un élément d'arbre de manière à former un liaison glissière entre le corps de support et l'élément d'arbre. L'élément d'arbre est rétractable pour permettre de placer et d'enlever le support. De manière préférentielle, il est prévu plusieurs supports de pièce à fabriquer interchangeables au sein de la machine.

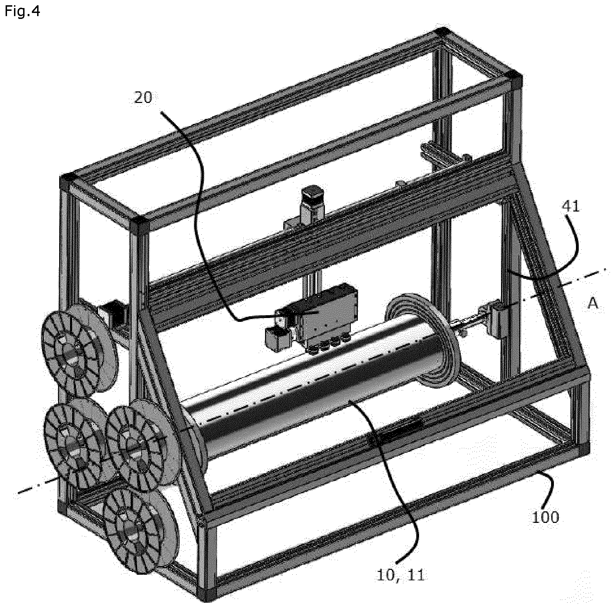

Selon un deuxième mode de réalisation représenté en figure 4, le support 10 présente une surface périphérique 11 circulaire.

On va maintenant décrire un procédé de fabrication par impression 3D par dépôt de filament en matière plastique fondu mis en oeuvre par la machine précédemment décrite. Le procédé prévoit les étapes suivantes :
- fournir au moins un filament en matière plastique par le dispositif d'alimentation (30),
- chauffer l'au moins un filament en matière plastique par l'élément chauffant de manière à dépasser la température de fusion dudit filament afin d'obtenir un filament en matière plastique fondu,
- commander la tête d'impression (20) pour déposer l'au moins un filament en matière plastique fondu sur une surface périphérique (11) du support (10) de pièce à fabriquer,
- mettre en rotation ledit support (10) et mettre en mouvement de translation selon une direction horizontale la tête d'impression (20) et/ou mettre en mouvement de translation selon une direction verticale la tête d'impression (20) par rapport au support (10) de pièce de façon à déposer l'au moins un filament en matière plastique fondu selon une trajectoire prédéterminée sur la surface périphérique 11 du support (10).

La répétition des précédentes étapes forme ainsi une pièce bidirectionnelle portée par la périphérie du support (10) de pièce.

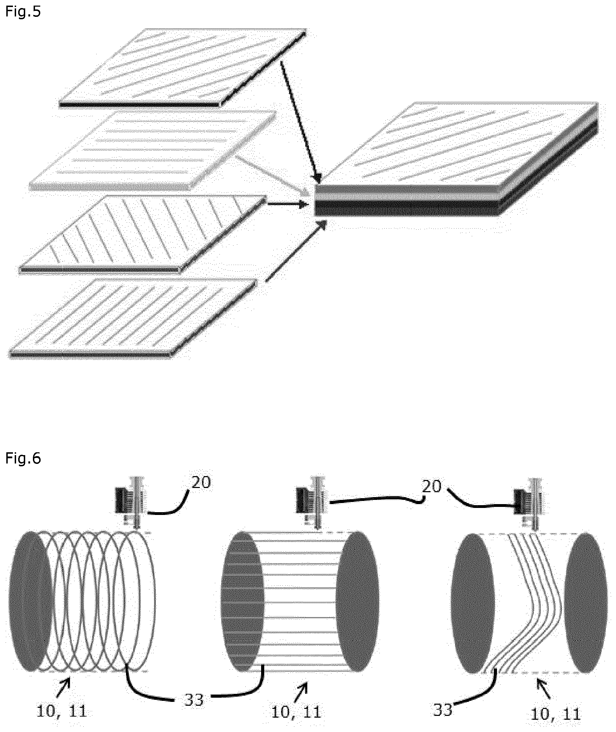

En références à la figure 6, les étapes de mise en rotation du support et de mise en mouvement de la tête d'impression sont des étapes successives ou simultanées permettant de disposer ou d'orienter les filaments dans des directions tangentielles (partie gauche de la figure 6), longitudinales (milieu de la figure 6) ou particulières du type obliques ou diagonales (partie droite de la figure 6). Il est alors possible de former des couches dont la disposition des filaments diffère entre chaque couche (illustrée par la figure 5), permettant de réaliser des pièces présentant des propriétés mécaniques localement différentes. Or dans un repère cartésien, il n'est pas possible (ou du moins plus difficile) de disposer des filaments dans des directions obliques ou diagonales lorsque la pièce à fabriquer est obtenue par empilement vertical des couches de filaments.

De préférence, le procédé prévoit de fournir quatre filaments en matière plastique, chaque filament présentant un matériau d'une composition particulière. Les filaments sont déposés l'un après l'autre lorsqu'il est nécessaire d'utiliser différents types de filaments.

En premier lieu, le procédé de fabrication prévoit de fournir et déposé un filament de matériau provisoire ou sacrificiel de façon à réaliser une surface de support correspondant à la forme extérieure de la paroi intérieure de la pièce à fabriquer. Cette première couche sera éliminée avec un simple passage d'eau, sans action d'ébavurage. Le matériau provisoire est par exemple de l'alcool poly(vinylique) dit « PVA ».

Les trois autres filaments sont constitués du même matériau d'acide poly(lactique) dit « PLA », mais de différentes compositions ou grades. Les trois filaments sont chacun constitué du matériau suivant :
- du PLA classique qui servira de base,
- du PLA renforcé qui constituera des parties plus rigides comme des renforts, et
- du PLA flexible, par exemple un mélange PLA/élastomère ou un mélange PLA/PCL, qui permettra d'avoir des parties plus souples.

L'intérêt de réaliser des mélanges avec le PLA est de conserver une compatibilité chimique de toute la structure, favorisant le phénomène de coalescence et donc l'adhésion inter-couches entre les matériaux.

Le choix des filaments chacun d'un matériau spécifique et leur disposition sur le support 10 permet de créer un gradient de propriétés mécaniques dans la structure de la pièce à fabriquer.

Le PLA a en outre comme avantage d'être compatible avec le PVA. L'utilisation de ce couple PLA/PVA permet à la pièce imprimée obtenue (par exemple un corset médical) de ne pas avoir de zones à ébavurer et donc un meilleur aspect de surface, en particulier au touché ce qui est un avantage lorsque la pièce obtenue est en contact avec le corps humain.

Par exemple la pièce obtenue présente une épaisseur de 5 millimètres.

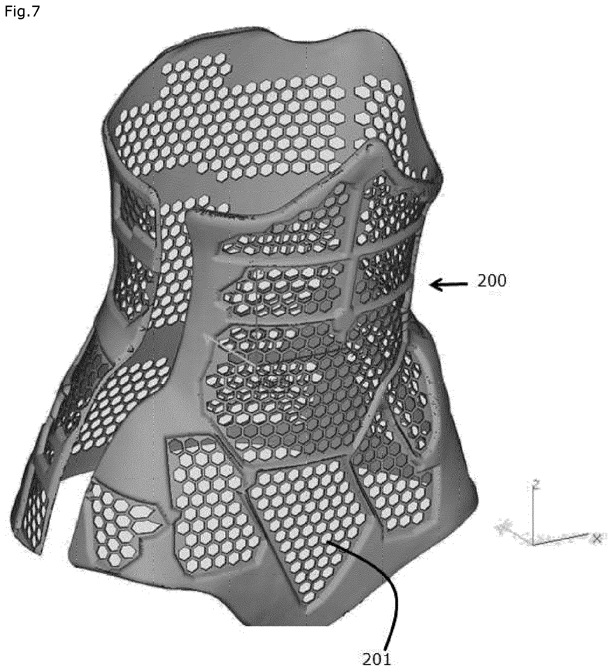

Le procédé de fabrication est en particulier utilisé pour fabriquer un corset médical représenté en figure 7. Après études par éléments finis via l'utilisation d'un logiciel dédié à cet effet, il est ainsi possible de fabriquer un corset médical d'une seule pièce qui comprend une ou plusieurs zones de renfort ou de correction de la posture du dos, lorsque ledit corset est porté par un patient. Il n'est donc pas nécessaire d'ajouter une pièce de renfort, par exemple métallique, le long du corset.

Au cours du procédé de fabrication, le ou les filaments peuvent être déposés selon une trajectoire prédéterminée sur le support 10 de manière à réaliser une pièce présentant un empilement de couches de filaments juxtaposés, chaque couche présentant une orientation générale des filaments différente de la couche précédente (voir partie droite de la figure 6). Cette caractéristique permet de réaliser une structure de pièce anisotrope.

De plus, grâce à l'agencement de couches de filament d'orientation variée, par exemple en quinconce, le corset obtenu est plus léger, tout en présentant des propriétés mécaniques localement différentes nécessaires au traitement thérapeutique. Le procédé permet en outre de faciliter la fabrication des structures du type treillis 201 entre des zones pleines.

Le procédé de fabrication présente ainsi les avantages suivants :
- la pièce obtenue présente des propriétés mécaniques localement différentes : par variations de matériaux, variations d'épaisseur de matériau(x), variation de la densité du matériau (à épaisseur donnée),
- les propriétés mécaniques qui différent entre des régions différentes sont de préférence réalisées de façon à présenter une variation continue et progressive entre ces régions (variation continue des paramètres, épaisseur, matériau, densité), ce améliore le confort et limite les risques de marques ou escarres,
- la pièce obtenue peut présenter un agencement en treillis ou dit « nid d'abeilles » plus ou moins dense, de très écarté à contigu à la limite d'un matériau plein, de façon à réaliser une aération ; l'épaisseur du treillis est par exemple de 2 millimètres.
- l'utilisation de treillis permet aussi une meilleure ventilation, même dans les régions à soutenir (et donc qui nécessitent la présence d'un matériau)
- la pièce obtenue peut présenter une continuité de matière même dans les régions sans soutien (ce qui permet d'y obtenir une simple rétention, c'est à dire sans appui correcteur mais permettant d'éviter les déplacements de graisses et les marques de bords).

Grâce à tous ces avantages, le corset médical obtenu est plus confortable, plus compacte de sorte qu'il est davantage toléré par le patient qui le porte.

Bien sûr, l'invention n'est pas limitée aux exemples qui viennent d'être décrits et de nombreux aménagements peuvent être apportés à ces exemples sans sortir du cadre de l'invention.

## Revendications

1. Machine (1) pour impression 3D par dépôt de filament en matière plastique fondu, comprenant :
- un bâti (100),
- un dispositif d'alimentation (30) d'au moins un filament en matière plastique,
- une tête d'impression (20) distribuant l'au moins un filament en matière plastique, ladite tête (20) comprenant au moins un élément chauffant de manière à chauffer l'au moins un filament en matière plastique au-dessus de sa température de fusion,
- des moyens de guidage (40) selon au moins un mouvement de translation horizontale de la tête d'impression (20) relativement au bâti (100), ledit dispositif d'alimentation (30) et ladite tête d'impression (20) étant agencés et configurés pour alimenter et distribuer au moins deux filaments de matières plastiques, chacun présentant des propriétés mécaniques différentes, dans lequel les matériaux des différents filaments comprennent au moins un matériau support provisoire et un ou plusieurs matériaux constituant la pièce à fabriquer, comprenant au moins un support (10) de pièce à fabriquer, ledit au moins un support (10) présentant un axe de rotation (A) s'étendant horizontalement et une surface périphérique (11) s'étendant autour de l'axe de rotation (A), de façon que l'au moins un filament est déposé sur la surface périphérique (11) du support (10) de pièce, la surface périphérique (11) présentant une forme elliptique selon sa section transversale, et s'évasant près d'une extrémité (12) de ladite surface selon sa section longitudinale, et comprenant un moyen de rotation (50) du support (10) de pièce de façon que ledit au moins un support (10) de pièce est rotatif selon son axe de rotation (A) par rapport au bâti (100).

2. Machine (1) selon la revendication 1, dans laquelle l'au moins un support (10) comprend plusieurs supports, interchangeables au sein de ladite machine, présentant des dimensions radiales différentes les uns par rapport aux autres.

3. Machine (1) selon la revendication 1 ou 2, dans laquelle l'au moins un support (10) est un cylindre.

4. Machine (1) selon l'une des revendications précédentes, dans laquelle l'au moins un support (10) comprend un organe chauffant.

5. Machine (1) selon l'une des revendications précédentes, dans laquelle la tête d'impression (20) comprend au moins deux buses (22) disposées de manière espacée les unes par rapport aux autres, la machine comprenant au moins deux moyens de guidage (40), chaque moyen de guidage étant associé à une seule buse, de manière que chaque buse puisse se déplacer indépendamment des autres buses.

6. Machine (1) selon l'une des revendications précédentes, dans laquelle la tête d'impression (20) comprend quatre buses (22) agencées et configurées pour distribuer quatre filaments de matières plastiques différentes.

7. Procédé de fabrication par impression 3D par dépôt de filament en matière plastique fondu, mis en oeuvre par une machine (1) pour impression 3D du type comprenant un bâti (100) portant :
- un support (10) de pièce à fabriquer, ledit support (10) présentant un axe de rotation (A) s'étendant horizontalement et une surface périphérique (11) s'étendant autour de l'axe de rotation (A), la surface périphérique (11) présentant une forme elliptique selon sa section transversale et s'évasant près d'une extrémité (12) de ladite surface selon sa section longitudinale,
- un dispositif d'alimentation (30) d'au moins un filament en matière plastique,
- une tête d'impression (20) distribuant l'au moins un filament en matière plastique, ladite tête comprenant au moins un élément chauffant de manière à chauffer l'au moins un filament en matière plastique au-dessus de sa température de fusion,
- des moyens de guidage (40) de la tête d'impression (20) relativement au bâti (100), agencés pour déposer sélectivement ledit filament en matière plastique fondu sur une surface du support (10) de pièce, et réaliser ainsi une fabrication additive sur ledit support (10) de pièce, ladite machine comprenant un dispositif d'alimentation (30) et une tête d'impression (20) agencés et configurés pour alimenter et distribuer au moins deux filaments de matières plastiques, chacun présentant des propriétés mécaniques différentes, les matériaux des différents filaments comprenant au moins un matériau support provisoire et un ou plusieurs matériaux constituant la pièce à fabriquer, le procédé comprenant les étapes suivantes :
- fournir au moins deux filaments en matière plastique par le dispositif d'alimentation (30),
- chauffer les au moins deux filaments en matière plastique par l'élément chauffant de manière à dépasser la température de fusion dudit filament afin d'obtenir des filaments en matière plastique fondu,
- commander la tête d'impression (20) pour déposer les au moins deux filaments en matière plastique fondu sur une surface périphérique (11) du support (10) de pièce,
- mettre en rotation ledit support (10) et mettre en mouvement de translation selon une direction horizontale la tête d'impression (20) par rapport au support (10) de pièce, de façon déposer successivement les au moins deux filaments en matière plastique fondu selon une trajectoire prédéterminée sur le support (10) de manière à réaliser une pièce présentant des propriétés mécaniques localement différentes, les filaments étant déposés selon une trajectoire prédéterminée sur le support (10), formant ainsi une pièce bidirectionnelle portée par ladite surface périphérique du support (10) de pièce et qui entoure son axe de rotation.

8. Procédé selon la revendication précédente, dans lequel on met en mouvement de translation selon une direction verticale la tête d'impression (20) par rapport au support (10) de pièce.

9. Procédé selon la revendication 7 ou 8, dans lequel on dépose lesdits au moins deux filaments en matière plastique fondu selon une trajectoire prédéterminée sur le support (10) de manière à réaliser une pièce présentant des propriétés mécaniques localement différentes.

10. Procédé selon l'une des revendications 7 à 9, dans lequel lesdits au moins deux filaments en matière plastique fondu sont déposés selon une trajectoire prédéterminée sur le support (10) de manière à réaliser une pièce présentant au moins une portion en forme de treillis agencé pour présenter des propriétés mécaniques déterminées localement.

11. Procédé selon l'une des revendications 7 à 10, dans lequel lesdits au moins deux filaments en matière plastique fondu sont déposés selon une trajectoire prédéterminée sur le support (10) de manière à réaliser une pièce présentant une épaisseur variable le long de sa périphérie et/ou selon sa direction longitudinale, de façon à présenter des propriétés mécaniques déterminées localement.

12. Procédé selon l'une des revendications 7 à 11, dans lequel, lesdits au moins deux filaments en matière plastique fondu sont déposés selon une trajectoire prédéterminée sur le support (10) de manière à réaliser une pièce présentant un empilement de couches de filaments juxtaposés, chaque couche présentant une orientation générale des filaments différente de la couche précédente.

13. Procédé selon l'une des revendications 7 à 12, dans lequel, lorsque la machine comprend un dispositif d'alimentation (30) et une tête d'impression (20) agencés et configurés pour alimenter et distribuer quatre filaments de matières plastiques, chacun présentant des propriétés mécaniques différentes, on dépose les quatre filaments fondus selon un ordre préétabli et selon une trajectoire prédéterminée sur le support (10) de manière à réaliser une pièce présentant des propriétés mécaniques localement différentes.

14. Procédé selon la revendication 7 ou 13, dans lequel les matériaux des différents filaments sont choisis parmi du PVA, PLA d'une première composition, PLA d'une seconde composition et PLA d'une troisième composition.

15. Procédé selon l'une des revendications 7 à 14, dans lequel la pièce fabriquée est un corset médical (200).

## Patentansprüche

1. Maschine (1) für den 3D-Druck durch Ablegen von Filament aus geschmolzenem Kunststoffmaterial, die das Folgende enthält:
- ein Gestell (100),
- eine Zuführvorrichtung (30) zum Zuführen von zumindest einem Kunststofffilament,
- einen Druckkopf (20), der das zumindest eine Kunststofffilament ausgibt, wobei der Kopf (20) zumindest ein Heizelement umfasst, um das zumindest eine Kunststofffilament über seine Schmelztemperatur zu erhitzen,
- Führungsmittel (40) zum Führen des Druckkopfs (20) bei zumindest einer horizontalen Translationsbewegung relativ zum Gestell (100),
wobei die Zuführvorrichtung (30) und der Druckkopf (20) dazu angeordnet und ausgelegt sind, zumindest zwei Kunststofffilamente mit jeweils verschiedenen mechanischen Eigenschaften zuzuführen und auszugeben,
wobei die Materialien der verschiedenen Filamente zumindest ein vorläufiges Trägermaterial und ein oder mehrere das herzustellende Werkstück bildende Materialien umfassen,
enthaltend zumindest eine Halterung (10) für das herzustellende Werkstück umfasst, wobei die zumindest eine Halterung (10) eine sich horizontal erstreckende Drehachse (A) und eine sich um die Drehachse (A) erstreckende Umfangsfläche (11) aufweist, so dass das zumindest eine Filament auf der Umfangsfläche (11) der Werkstückhalterung (10) abgelegt wird, wobei die Umfangsfläche (11) im Querschnitt eine elliptische Form aufweist und sich nahe bei einem Ende (12) der Fläche im Längsschnitt erweitert,
enthaltend eine Dreheinrichtung (50) zum Drehen der Werkstückhalterung (10), so dass die zumindest eine Werkstückhalterung (10) um ihre Drehachse (A) in Bezug auf das Gestell (100) drehbar ist.

2. Maschine (1) nach Anspruch 1, wobei die zumindest eine Halterung (10) mehrere Halterungen umfasst, die innerhalb der Maschine austauschbar sind und zueinander unterschiedliche radiale Abmessungen aufweisen.

3. Maschine (1) nach Anspruch 1 oder 2, wobei die zumindest eine Halterung (10) ein Zylinder ist.

4. Maschine (1) nach einem der vorhergehenden Ansprüche, wobei die zumindest eine Halterung (10) eine Heizeinrichtung umfasst.

5. Maschine (1) nach einem der vorhergehenden Ansprüche, wobei der Druckkopf (20) zumindest zwei Düsen (22) umfasst, die beabstandet zueinander angeordnet sind, wobei die Maschine zumindest zwei Führungsmittel (40) umfasst, wobei jedes Führungsmittel einer einzelnen Düse zugeordnet ist, so dass sich jede Düse unabhängig von den anderen Düsen bewegen kann.

6. Maschine (1) nach einem der vorhergehenden Ansprüche, wobei der Druckkopf (20) vier Düsen (22) umfasst, die dazu angeordnet und ausgelegt sind, vier Filamente aus unterschiedlichen Kunststoffmaterialien auszugeben.

7. 3D-Druck-Herstellungsverfahren durch Ablegen von Filament aus geschmolzenem Kunststoffmaterial, durchgeführt von einer Maschine (1) für den 3D-Druck vom Typ umfassend ein Gestell (100), das das Folgende trägt:
- eine Halterung (10) für das herzustellende Werkstück, wobei die Halterung (10) eine sich horizontal erstreckende Drehachse (A) und eine sich um die Drehachse (A) erstreckende Umfangsfläche (11) aufweist, wobei die Umfangsfläche (11) im Querschnitt eine elliptische Form aufweist und sich nahe bei einem Ende (12) der Fläche im Längsschnitt erweitert,
- eine Zuführvorrichtung (30) zum Zuführen von zumindest einem Kunststofffilament,
- einen Druckkopf (20), der das zumindest eine Kunststofffilament ausgibt, wobei der Kopf zumindest ein Heizelement umfasst, um das zumindest eine Kunststofffilament über seine Schmelztemperatur zu erhitzen,
- Führungsmittel (40) zum Führen des Druckkopfs (20) relativ zum Gestell (100), die dazu angeordnet sind, das geschmolzene Kunststofffilament selektiv auf einer Fläche der Werkstückhalterung (10) abzulegen und dadurch eine additive Fertigung auf der Werkstückhalterung (10) durchzuführen,
wobei die Maschine eine Zuführvorrichtung (30) und einen Druckkopf (20) enthält, die dazu angeordnet und ausgelegt sind, zumindest zwei Kunststofffilamente mit jeweils verschiedenen mechanischen Eigenschaften zuzuführen und auszugeben, wobei die Materialien der verschiedenen Filamente zumindest ein vorläufiges Trägermaterial und ein oder mehrere das herzustellende Werkstück bildende Materialien umfassen,
wobei das Verfahren die folgenden Schritte umfasst:
- Bereitstellen von zumindest zwei Kunststofffilamenten durch die Zuführvorrichtung (30),
- Erhitzen der zumindest zwei Kunststofffilamente durch das Heizelement, so dass die Schmelztemperatur des Filaments überschritten wird, um Filamente aus geschmolzenem Kunststoffmaterial zu erhalten,
- Ansteuern des Druckkopfs (20), um die zumindest zwei Filamente aus geschmolzenem Kunststoffmaterial auf einer Umfangsfläche (11) der Werkstückhalterung (10) abzulegen,
- Drehen der Halterung (10) und Verschieben des Druckkopfs (20) in einer horizontalen Richtung relativ zur Werkstückhalterung (10), um nacheinander die zumindest zwei Filamente aus geschmolzenem Kunststoffmaterial entlang einer vorbestimmten Bahn auf der Halterung (10) abzulegen, so dass ein Werkstück mit bereichsweise verschiedenen mechanischen Eigenschaften ausgebildet wird, wobei die Filamente entlang einer vorbestimmten Bahn auf der Halterung (10) abgelegt werden und dadurch ein bidirektionales Werkstück bilden, das von der Umfangsfläche der Werkstückhalterung (10) getragen wird und deren Drehachse umgibt.

8. Verfahren nach dem vorhergehenden Anspruch, wobei der Druckkopf (20) in Bezug auf die Werkstückhalterung (10) in vertikaler Richtung verschoben wird.

9. Verfahren nach Anspruch 7 oder 8, wobei die zumindest zwei Filamente aus geschmolzenem Kunststoffmaterial entlang einer vorbestimmten Bahn auf der Halterung (10) abgelegt werden, um ein Werkstück mit bereichsweise verschiedenen mechanischen Eigenschaften auszubilden.

10. Verfahren nach einem der Ansprüche 7 bis 9, wobei die zumindest zwei Filamente aus geschmolzenem Kunststoffmaterial entlang einer vorbestimmten Bahn auf der Halterung (10) abgelegt werden, um ein Werkstück mit zumindest einem gitterförmigen Abschnitt auszubilden, der so angeordnet ist, dass er bereichsweise bestimmte mechanische Eigenschaften aufweist.

11. Verfahren nach einem der Ansprüche 7 bis 10, wobei die zumindest zwei Filamente aus geschmolzenem Kunststoffmaterial entlang einer vorbestimmten Bahn auf der Halterung (10) abgelegt werden, um ein Werkstück mit einer entlang seines Umfangs und/oder in seiner Längsrichtung variablen Dicke auszubilden, so dass es bereichsweise bestimmte mechanische Eigenschaften aufweist.

12. Verfahren nach einem der Ansprüche 7 bis 11, wobei die zumindest zwei Filamente aus geschmolzenem Kunststoffmaterial entlang einer vorbestimmten Bahn auf der Halterung (10) abgelegt werden, um ein Werkstück mit einer Stapelung von Schichten aus nebeneinander liegenden Filamenten auszubilden, wobei jede Schicht eine Filamentausrichtung aufweist, die sich von der vorhergehenden Schicht unterscheidet.

13. Verfahren nach einem der Ansprüche 7 bis 12, wobei dann, wenn die Maschine eine Zuführvorrichtung (30) und einen Druckkopf (20) umfasst, die dazu angeordnet und ausgelegt sind, zumindest vier Kunststofffilamente mit jeweils verschiedenen mechanischen Eigenschaften zuzuführen und auszugeben, die vier geschmolzenen Filamente in einer vorgegebenen Reihenfolge und entlang einer vorbestimmten Bahn auf der Halterung (10) abgelegt werden, um ein Werkstück mit bereichsweise verschiedenen mechanischen Eigenschaften herzustellen.

14. Verfahren nach einem der Ansprüche 7 oder 13, wobei die Materialien der verschiedenen Filamente ausgewählt sind aus PVA, PLA einer ersten Zusammensetzung, PLA einer zweiten Zusammensetzung und PLA einer dritten Zusammensetzung.

15. Verfahren nach einem der Ansprüche 7 bis 14, wobei es sich bei dem hergestellten Werkstück um ein medizinisches Korsett (200) handelt.

## Claims

1. Machine (1) for 3D printing by deposition of melted filament of plastic material, comprising:
- a machine frame (100),
- a device (30) for feeding at least one filament of plastic material,
- a print head (20) distributing the at least one filament of plastic material, said head (20) comprising at least one heating element so as to heat the at least one filament of plastic material above its melting temperature,
- means (40) for guiding according to at least one horizontal translational movement of the print head (20) in relation to the machine frame (100), said feed device (30) and said print head (20) being arranged and configured to feed and distribute at least two filaments of plastic materials, each having different mechanical properties,
in which the materials of the different filaments comprise at least one temporary support material and one or more materials constituting the workpiece,
comprising at least one workpiece support (10), said at least one support (10) having an axis of rotation (A) extending horizontally and a peripheral surface (11) extending about the axis of rotation (A), such that the at least one filament is deposited on the peripheral surface (11) of the workpiece support (10), the peripheral surface (11) having an elliptical shape along its transverse section, and flaring close to an end (12) of said surface along its longitudinal section,
and comprising a rotation means (50) of the workpiece support (10) such that said at least one workpiece support (10) is rotary about its axis of rotation (A) with respect to the machine frame (100).

2. Machine (1) according to claim 1, in which the at least one support (10) comprises several supports, interchangeable within said machine, having different radial dimensions with respect to one another.

3. Machine (1) according to claim 1 or 2, in which the at least one support (10) is a cylinder.

4. Machine (1) according to one of the preceding claims, in which the at least one support (10) comprises a heating component.

5. Machine (1) according to one of the preceding claims, in which the print head (20) comprises at least two nozzles (22) placed so as to be spaced apart from one another, the machine comprising at least two guide means (40), each guide means being associated with a single nozzle, so that each nozzle can be displaced independently from the other nozzles.

6. Machine (1) according to one of the preceding claims, in which the print head (20) comprises four nozzles (22) arranged and configured to distribute four filaments of different plastic materials.

7. Method of manufacturing by 3D printing by deposition of melted filament of plastic material, implemented by a machine (1) for 3D printing of the type comprising a machine frame (100) bearing:
- a workpiece support (10), said support (10) having an axis of rotation (A) extending horizontally and a peripheral surface (11) extending about the axis of rotation (A), the peripheral surface (11) having an elliptical shape along its transverse section, and flaring close to an end (12) of said surface along its longitudinal section,
- a device (30) for feeding at least one filament of plastic material,
- a print head (20) distributing the at least one filament of plastic material, said head comprising at least one heating element so as to heat the at least one filament of plastic material above its melting temperature,
- means (40) for guiding the print head (20) in relation to the machine frame (100), arranged to selectively deposit said melted filament of plastic material on a surface of the workpiece support (10), and thus carry out additive manufacturing on said workpiece support (10),
said machine comprising a feed device (30) and a print head (20) arranged and configured to feed and distribute at least two filaments of plastic materials, each having different mechanical properties, the materials of the different filaments comprising at least one temporary support material and one or more materials constituting the workpiece,
the method comprising the following steps:
- supplying at least two filaments of plastic material by means of the feed device (30),
- heating the at least two filaments of plastic material by the heating element so as to exceed the melting temperature of said filament in order to obtain melted filaments of plastic material,
- driving the print head (20) to deposit the at least two melted filaments of plastic material on a peripheral surface (11) of the workpiece support (10),
- rotating said support (10) and moving the print head (20) in translation in a horizontal direction with respect to the workpiece support (10), so as to deposit successively the at least two melted filaments of plastic material along a predetermined path on the support (10) so as to produce a piece having mechanical properties that are locally different, the filaments being deposited along a predetermined path on the support (10), thus forming a bidirectional piece borne by said peripheral surface of the workpiece support (10) and which surrounds its axis of rotation.

8. Method according to the preceding claim, in which the print head is moved in translation in a vertical direction (20) with respect to the workpiece support (10).

9. Method according to claim 7 or 8, in which said at least two melted filaments of plastic material are deposited along a predetermined path on the support (10) so as to produce a piece having mechanical properties that are locally different.

10. Method according to one of claims 7 to 9, in which said at least two melted filaments of plastic material are deposited along a predetermined path on the support (10) so as to produce a piece having at least one portion in the form of a mesh arranged to have mechanical properties that are locally determined.

11. Method according to one of claims 7 to 10, in which said at least two melted filaments of plastic material are deposited along a predetermined path on the support (10) so as to produce a piece having a variable thickness along its periphery and/or along its longitudinal direction, such as to have mechanical properties that are locally determined.

12. Method according to one of claims 7 to 11, in which said at least two melted filaments of plastic material are deposited along a predetermined path on the support (10) so as to produce a piece having a stack of layers of juxtaposed filaments, each layer having a general orientation of the filaments that is different to the preceding layer.

13. Method according to one of claims 7 to 12, in which, when the machine comprises a feed device (30) and a print head (20) arranged and configured to feed and distribute four filaments of plastic materials, each having different mechanical properties, the four melted filaments are deposited in a pre-established order and along a predetermined path on the support (10) so as to produce a piece having mechanical properties that are locally different.

14. Method according to claim 7 or 13, in which the materials of the different filaments are selected from PVA, PLA of a first composition, PLA of a second composition and PLA of a third composition.

15. Method according to one of claims 7 to 14, in which the manufactured piece is an orthopaedic girdle (200).
